# EUROPÄISCHE PATENTANMELDUNG

(11) **EP 4 549 432 A1**
(43) Veröffentlichungstag der Anmeldung: **07.05.2025**
(21) Anmeldenummer: 23206882.5
(22) Anmeldetag: 31.10.2023
(51) Int. Cl.: C07D 271/113, A01N 43/82

(54) **THERMODYNAMISCH STABILE KRISTALLMODIFIKATION VON 2-CHLOR-N-(5-METHYL-1,3,4-OXADIAZOL-2-YL)-3-[(S)-METHYLSULFINYL]-4-(TRIFLUORMETHYL)BENZAMID**

(71) Anmelder: Bayer Aktiengesellschaft, 51373 Leverkusen (DE)
(72) Erfinder: Die Erfindernennung liegt noch nicht vor
(74) Vertreter: BIP Patents

(57) **Zusammenfassung**

Es wird eine thermodynamisch stabile Kristallmodifikation des herbiziden Wirkstoffs 2-Chlor-N-(5-methyl-1,3,4-oxadiazol-2-yl)-3-[(*S*)-methylsulfinyl]-4-(trifluormethyl)benzamid beschrieben. Diese thermodynamisch stabile Kristallmodifikation weist besondere Vorteile in der Stabilität von Suspensionformulierungen auf.

## Beschreibung

Die Erfindung betrifft das technische Gebiet der Pflanzenschutzmittel.

Spezieller betrifft sie die thermodynamisch stabile Kristallmodifikation von 2-Chlor-N-(5-methyl-1,3,4-oxadiazol-2-yl)-3-[(*S*)-methylsulfinyl]-4-(trifluormethyl)benzamid der Formel (I)

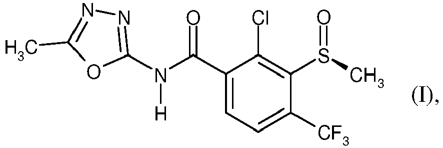

Sowie Verfahren zu ihrer Herstellung und deren Verwendung als Herbizid. Die Verbindung der Formel (I) wird im Folgenden unabhängig von ihrer Erscheinungsform als "Benzamid" bezeichnet.

Es ist bekannt, dass einige organische Verbindungen in nur einer Kristallstruktur, andere hingegen, sogenannte Polymorphe, in verschiedenen Kristallstrukturen auftreten können, siehe dazu beispielsweise J. Bernstein, R.J. Davey, J.O. Henck, Angew. Chem. Int. Ed., 1999, 38, 3440-3461. So sind aus EP 1 314 724 A1 zwei Kristallstrukturen des herbiziden Wirkstoffs Sulcotrione bekannt.

Das unter der CAS-Nr. 2833716-54-2 und auch aus WO2022/189495 A1 (dort Beispiel Nr. I-5) bekannte Benzamid besitzt herbizide Eigenschaften und eignet sich für die Herstellung von Pflanzenschutzmitteln, die zur Unkrautbekämpfung herangezogen werden.

Aufgabe der vorliegenden Erfindung bestand in der Bereitstellung einer stabilen Kristallmodifikation von 2-Chlor-N-(5-methyl-1,3,4-oxadiazol-2-yl)-3-[(S)-methylsulfinyl]-4-(trifluormethyl)benzamid. Diese Aufgabe wurde erfindungsgemäß gelöst.

Ein Gegenstand der Erfindung ist daher eine thermodynamisch stabile Kristallmodifikation des Benzamids 2-Chlor-N-(5-methyl-1,3,4-oxadiazol-2-yl)-3-[(*S*)-methylsulfinyl]-4-(trifluormethyl)benzamid.

Die Begriffe "Modifikation" und "Kristallmodifikation" werden im Folgenden als gleichbedeutend verstanden.

Die erfindungsgemäße stabile Kristallmodifikation weist ein charakteristisches Raman-Spektrum auf, das in nachfolgender Tabelle dargestellt ist, wobei die Werte der Bandenmaxima in Wellenzahlen und deren Intensitäten angegeben sind.

### Messbedingungen:

| Gerät | Bruker Raman RFS 100/S |
|---|---|
| Anzahl Scans | 64 |
| Auflösung | 2 cm⁻¹ |
| Laser Power | 50 mW |
| Laser Wellenlänge | 1064 nm |

**Tabelle 1: Bandenmaxima der Raman-Spektren [cm⁻¹] und Intensität**

| **Position** | | **Intensity** | |
|---|---|---|---|
| | 97,08 | | 126,927 |
| | 118,43 | | 39,233 |
| | 142,91 | | 44,800 |
| | 170,64 | | 37,407 |
| | 83,49 | | 46,218 |
| | 225,96 | | 29,391 |
| | 248,10 | | 24,920 |
| | 272,87 | | 16,545 |
| | 288,37 | | 16,900 |
| | 301,29 | | 17,613 |
| | 348,57 | | 19,837 |
| | 364,26 | | 16,905 |
| | 386,96 | | 15,128 |
| | 428,87 | | 18,588 |
| | 465,06 | | 39,770 |
| | 528,95 | | 14,149 |
| | 572,95 | | 14,623 |
| | 596,96 | | 15,375 |
| | 680,03 | | 35,370 |
| | 706,53 | | 12,394 |
| | 728,81 | | 12,879 |
| | 834,86 | | 9,222 |
| | 904,28 | | 13,519 |
| | 959,11 | | 18,749 |
| | 1050,94 | | 30,399 |
| | 1061,51 | | 29,155 |
| | 1101,59 | | 19,954 |
| | 1177,10 | | 16,196 |
| | 1265,45 | | 14,642 |
| | 1282,95 | | 14,388 |
| | 1304,18 | | 15,002 |
| | 1361,83 | | 11,711 |
| | 1409,10 | | 12,803 |
| | 1555,81 | | 20,298 |
| | 1589,71 | | 22,125 |
| | 1613,93 | | 61,040 |
| | 1707,09 | | 36,799 |
| | 2928,94 | | 53,499 |
| | 2972,24 | | 13,239 |
| | 3007,\|62 | | 22,215 |
| | 3065,20 | | 22,345 |
| | 3077,1 9 | | 19,548 |

Die Röntgen-Pulver-Diffraktometrie der stabilen Kristallmodifikation zeigt für diese Kristallmodifikation charakteristische Peaks, die in Tabelle 2 angegeben sind.

**Tabelle 2: Röntgen-Pulver-Diffraktometrie Muster**

| **Peak list** | | | | |
|---|---|---|---|---|
| **Pos. [°2Th.]** | **Height [cts]** | **FWHM [°2Th.]** | **d-spacing [Å]** | **Rel. Int. [%]** |
| 9,9232 | 151,36 | 0,0492 | 8,90645 | 12,07 |
| 10,9655 | 547,08 | 0,1378 | 8,06208 | 43,61 |
| 13,9003 | 403,91 | 0,0689 | 6,36581 | 32,20 |
| 14,7616 | 389,96 | 0,0787 | 5,99624 | 31,09 |
| 17,0624 | 569,06 | 0,1082 | 5,19253 | 45,37 |
| 17,7507 | 192,34 | 0,1181 | 4,99268 | 15,33 |
| 19,7251 | 244,27 | 0,0689 | 4,49718 | 19,47 |
| 20,3365 | 705,41 | 0,1082 | 4,36332 | 56,24 |
| 22,0352 | 465,76 | 0,0984 | 4,03065 | 37,13 |
| 22,6026 | 645,57 | 0,1378 | 3,93072 | 51,47 |
| 24,2061 | 1254,35 | 0,1181 | 3,67385 | 100,00 |
| 24,6826 | 182,24 | 0,1574 | 3,60400 | 14,53 |
| 26,6012 | 384,48 | 0,0787 | 3,34826 | 30,65 |
| 27,9726 | 699,49 | 0,0984 | 3,18714 | 55,77 |
| 28,4150 | 370,88 | 0,0720 | 3,13852 | 29,57 |
| 28,4373 | 375,35 | 0,0492 | 3,13610 | 29,92 |
| 29,7363 | 112,68 | 0,1181 | 3,00200 | 8,98 |
| 31,3232 | 378,16 | 0,0886 | 2,85344 | 30,15 |
| 31,7258 | 225,18 | 0,1378 | 2,81813 | 17,95 |
| 32,8716 | 181,77 | 0,0590 | 2,72248 | 14,49 |
| 33,2869 | 282,82 | 0,1771 | 2,68945 | 22,55 |
| 34,4228 | 121,61 | 0,0590 | 2,60326 | 9,69 |
| 34,7152 | 80,45 | 0,0984 | 2,58199 | 6,41 |
| 35,8917 | 38,06 | 0,1181 | 2,50001 | 3,03 |
| 36,2268 | 161,04 | 0,0984 | 2,47765 | 12,84 |
| 37,2805 | 206,59 | 0,0787 | 2,41001 | 16,47 |
| 37,5917 | 54,48 | 0,0590 | 2,39077 | 4,34 |
| 38,9793 | 76,61 | 0,0984 | 2,30879 | 6,11 |

### Messbedingungen:

| | |
|---|---|
| Scan Axis | Gonio |
| Start Position [°2Th.] | 2.0066 |
| End Position [°2Th.] | 37.9906 |
| Anode, Material | Cu |
| K-Alpha1 [Ä] | 1.54060 |
| K-Alpha2 [Ä] | 1.54443 |
| K-Beta [Ä] | 1.39225 |
| K-A2 / K-A1 Ratio | 0.50000 |
| Generator Settings | 40 mA, 40 kV |
| Incident Beam Monochromator | focusing x-xay mirror |
| Spinning | Yes |

Das Benzamid der Formel (I) an sich kann beispielsweise nach einem der in WO2022/189495, WO 2012/126932 und WO 2018/202535 genannten Verfahren hergestellt werden. In Abhängigkeit von der Art des im letzten Reinigungsschritt verwendeten Lösungsmittels und der Temperaturführung fällt das Benzamid üblicherweise in amorpher Form, in Form der hier beschriebenen stabilen Kristallmodifikation oder in einem Gemisch der amorphen Form und der stabilen Kristallmodifikation an.

Aufgrund ihrer Stabilität eignet sich die stabile Kristallmodifikation des Benzamids ganz allgemein als Ausgangsmaterial für die Herstellung jedweder dieses Benzamid enthaltender Pflanzenschutzformulierungen, auch wenn das Benzamid nach der Formulierung nicht mehr in dieser Form, sondern etwa in gelöster Form vorliegt.

Gegenstand der Erfindung sind daher auch Verfahren zur Herstellung des Benzamids enthaltenden Pflanzenschutzformulierungen, welche die stabile Kristallmodifikation II des Benzamids verwenden sowie dieses Benzamid enthaltende Pflanzenschutzformulierungen, die aus der stabilen Kristallmodifikation II des Benzamids erhalten wurden. Durch den Einsatz der stabilen Kristallmodifikation II wird die Sicherheit für Zubereitungen des Benzamids erhöht und somit das Risiko falscher Dosierungen verringert.

Die stabile Kristallmodifikation des Benzamids kann in bekannter Weise in die üblichen Formulierungen überführt werden, wie Suspensionskonzentrate, kolloidale Konzentrate, dispergierbare Konzentrate, emulgierbare Konzentrate (Emulsionskonzentrate), Emulsionsbeizen, Suspensionsbeizen, Granulate, Mikrogranulate, Suspoemulsionen, Öldispersionen, wasserlösliche Granulate, wasserlösliche Konzentrate und wasserdispergierbare Granulate, unter Verwendung geeigneter Hilfs- und Trägerstoffe oder Lösemittel. Hierbei soll die wirksame Verbindung in einer Konzentration von etwa 0,5 bis 90 Gew.-% der Gesamtmischung vorhanden sein, d.h. in Mengen, die ausreichend sind, um den notwendigen Dosierungsspiegel zu erreichen. Die Formulierungen werden beispielsweise hergestellt durch Verstrecken der stabilen Kristallmodifikation II des Benzamids mit Lösemitteln und/oder Trägerstoffen, gegebenenfalls unter Verwendung von Emulgier- und/oder Dispergiermitteln, und/oder anderen Hilfsstoffen, wie z.B. Penetrationshilfsmitteln.

Die Anwendung erfolgt in der üblichen Weise, indem die unerwünschten Pflanzen und/oder ihr Lebensraum mit dem Wirkstoff bzw. dessen Formulierung in Kontakt gebracht werden.

Das Benzamid in der stabilen Kristallmodifikation zeigt eine hervorragende herbizide Wirkung gegenüber Vertretern der Gruppe sowohl der monokotylen als auch der dikotylen Pflanzen. Beispielhaft seien hier genannt:
Dikotyle Pflanzen der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Monokotyle Pflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Gegenstand der Erfindung ist daher auch die Verwendung der stabilen Kristallmodifikation des Benzamids zur Herstellung eines Pflanzenschutzmittels zur Behandlung des Unkrautbefalls.

Die erfindungsgemäße stabile Kristallmodifikation II des Benzamids ist aufgrund ihrer hohen Verträglichkeit gegenüber Kulturpflanzen zur Bekämpfung unerwünschter Pflanzen in Kulturen von beispielsweise Weizen, Gerste, Hafer, Roggen, Reis, Mais, Zuckerrübe, Zuckerrohr, Baumwolle und Soja, insbesondere in Weizen, Gerste, Hafer und Roggen, geeignet.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit der erfindungsgemäßen Kristallmodifikation II des Benzamids erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen oder Aufstreichen.

Die erfindungsgemäße Kristallmodifikation II des Benzamids kann, wie bereits oben ausgeführt, in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent des Wirkstoffs in der erfindungsgemäßen Kristallmodifikation II, vorzugsweise zwischen 0,5 und 90 %.

Die erfindungsgemäße Kristallmodifikation des Benzamids kann als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, - meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesosulfuron (-methyl, -sodium), Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise AD-67, BAS-145138, Benoxacor, Cloquintocet (-mexyl), Cyometrinil, Cyprosulfamide, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die erfindungsgemäße Kristallmodifikation II des Benzamids kann als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäße Kristallmodifikation II des Benzamids kann sowohl vor als auch nach dem Auflaufen der Pflanzen appliziert werden. Sie kann auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 1 kg Wirkstoff pro Hektar Bodenfläche, vorzugsweise zwischen 5 g und 500 g pro ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die sowohl durch konventionelle Züchtung, durch Mutagenese oder durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel - auch in Kombination mit anderen agrochemischen Wirkstoffen - , besseres Pflanzenwachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Soja, Kartoffel, Baumwolle, Raps sowie insbesondere Mais sowie Obstpflanzen (mit den Früchten Äpfel, Birnen, Zitrusfrüchten und Weintrauben) erwähnt, wobei insbesondere Mais, aber auch Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus *Bacillus thuringiensis* (z.B. durch die Gene CrylA(a), CrylA(b), CryIA(c), CryIIA, CryIIIA, CryIIIB2, Cry9c, Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien v.a. Maissorten, jedoch ebenso Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid tolerante Pflanzen seien v.a. Maissorten, jedoch ebenso Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

## Patentansprüche

1. Thermodynamisch stabile Kristallmodifikation von 2-Chlor-N-(5-methyl-1,3,4-oxadiazol-2-yl)-3-[(S)-methylsulfinyl]-4-(trifluormethyl)benzamid, worin die Kristallmodifikation
a) ein Raman-Spektrum aufweist mit Bandenmaxima [cm⁻¹] von:
| **Position** | | **Intensity** | |
|---|---|---|---|
| | 97,08 | | 126,927 |
| | 118,43 | | 39,233 |
| | 142,91 | | 44,800 |
| | 170,64 | | 37,407 |
| | 183,49 | | 46,218 |
| | 225,96 | | 29,391 |
| | 248,10 | | 24,920 |
| | 272,87 | | 16,545 |
| | 288,37 | | 16,900 |
| | 301,29 | | 17,613 |
| | 348,57 | | 19,837 |
| | 364,26 | | 16,905 |
| | 386,96 | | 15,128 |
| | 428,87 | | 18,588 |
| | 465,06 | | 39,770 |
| | 528,95 | | 14,149 |
| | 572,95 | | 14,623 |
| | 596,96 | | 15,375 |
| | 680,03 | | 35,370 |
| | 706,53 | | 12,394 |
| | 728,81 | | 12,879 |
| | 834,86 | | 9,222 |
| | 904,28 | | 13,519 |
| | 959,11 | | 18,749 |
| | 1050,94 | | 30,399 |
| | 1061,51 | | 29,155 |
| | 1101,59 | | 19,954 |
| | 1177,10 | | 16,196 |
| | 1265,45 | | 14,642 |
| | 1282,95 | | 14,388 |
| | 1304,18 | | 15,002 |
| | 1361,83 | | 11,711 |
| | 1409,10 | | 12,803 |
| | 1555,81 | | 20,298 |
| | 1589,71 | | 22,125 |
| | 1613,93 | | 61,040 |
| | 1707,09 | | 36,799 |
| | 2928,94 | | 53,499 |
| | 2972,24 | | 13,239 |
| | 3007,62 | | 22,215 |
| | 3065,20 | | 22,345 |
| | 3077,19 | | 19,548 |
und
b) ein Röntgen-Pulver-Diffraktometrie Muster mit folgenden Peaks, angegeben in Grad 2 Theta, aufweist:
| **Pos. [°2Th.]** | **Height [cts]** | **FWHM [°2Th.]** | **d-spacing [Å]** | **Rel. Int. [%]** |
|---|---|---|---|---|
| 9,9232 | 151,36 | 0,0492 | 8,90645 | 12,07 |
| 10,9655 | 547,08 | 0,1378 | 8,06208 | 43,61 |
| 13,9003 | 403,91 | 0,0689 | 6,36581 | 32,20 |
| 14,7616 | 389,96 | 0,0787 | 5,99624 | 31,09 |
| 17,0624 | 569,06 | 0,1082 | 5,19253 | 45,37 |
| 17,7507 | 192,34 | 0,1181 | 4,99268 | 15,33 |
| 19,7251 | 244,27 | 0,0689 | 4,49718 | 19,47 |
| 20,3365 | 705,41 | 0,1082 | 4,36332 | 56,24 |
| 22,0352 | 465,76 | 0,0984 | 4,03065 | 37,13 |
| 22,6026 | 645,57 | 0,1378 | 3,93072 | 51,47 |
| 24,2061 | 1254,35 | 0,1181 | 3,67385 | 100,00 |
| 24,6826 | 182,24 | 0,1574 | 3,60400 | 1 4,53 |
| 26,6012 | 384,48 | 0,0787 | 3,34826 | 30,65 |
| 27,9726 | 699,49 | 0,0984 | 3,18714 | 55,77 |
| 28,4150 | 370,88 | 0,0720 | 3,13852 | 29,57 |
| 28,4373 | 375,35 | 0,0492 | 3,13610 | 29,92 |
| 29,7363 | 112,68 | 0,1181 | 3,00200 | 8,98 |
| 31,3232 | 378,16 | 0,0886 | 2,85344 | 30,15 |
| 31,7258 | 225,18 | 0,1378 | 2,81813 | 17,95 |
| 32,8716 | 181,77 | 0,0590 | 2,72248 | 14,49 |
| 33,2869 | 282,82 | 0,1771 | 2,68945 | 22,55 |
| 34,4228 | 121,61 | 0,0590 | 2,60326 | 9,69 |
| 34,7152 | 80,45 | 0,0984 | 2,58199 | 6,41 |
| 35,8917 | 38,06 | 0,1181 | 2,50001 | 3,03 |
| 36,2268 | 161,04 | 0,0984 | 2,47765 | 12,84 |
| 37,2805 | 206,59 | 0,0787 | 2,41001 | 16,47 |
| 37,5917 | 54,48 | 0,0590 | 2,39077 | 4,34 |
| 38,9793 | 76,61 | 0,0984 | 2,30879 | 6,11 |

2. Herbizides Mittel, **gekennzeichnet durch** einen Gehalt an der thermodynamischen stabilen Kristallmodifikation von 2-Chlor-N-(5-methyl-1,3,4-oxadiazol-2-yl)-3-[(*S*)-methylsulfinyl]-4-(trifluormethyl)benzamid gemäß Anspruch 1 und gängigen Streckmitteln und/oder oberflächenaktiven Hilfsstoffen.

3. Herbizides Mittel nach Anspruch 2 oder 3, **dadurch gekennzeichnet, dass** 2-Chlor-N-(5-methyl-1,3,4-oxadiazol-2-yl)-3-[(S)-methylsulfinyl]-4-(trifluormethyl)benzamid zu mehr als 90 Gew.-% in der stabilen Kristallmodifikation vorliegt

4. Herbizides Mittel nach Anspruch 3, **dadurch gekennzeichnet, dass** 2-Chlor-N-(5-methyl-1,3,4-oxadiazol-2-yl)-3-[(*S*)-methylsulfinyl]-4-(trifluormethyl)benzamid zu mehr als 95 Gew.-% in der stabilen Kristallmodifikation vorliegt.

5. Herbizides Mittel nach Anspruch 3, **dadurch gekennzeichnet, dass** das 2-Chlor-N-(5-methyl-1,3,4-oxadiazol-2-yl)-3-[(S)-methylsulfinyl]-4-(trifluormethyl)benzamid zu mehr als 98 Gew.-% in der stabilen Kristallmodifikation vorliegt.

6. Verwendung der thermodynamisch stabilen Kristallmodifikation von 2-Chlor-N-(5-methyl-1,3,4-oxadiazol-2-yl)-3-[(S)-methylsulfinyl]-4-(trifluormethyl)benzamid gemäß Anspruch 1 oder eines Mittels gemäß einem der Ansprüche 2 bis 5 zur Bekämpfung von unerwünschten Pflanzen.

7. Verfahren zur Bekämpfung von unerwünschten Pflanzen, **dadurch gekennzeichnet, dass** man die thermodynamisch stabile Kristallmodifikation von 2-Chlor-N-(5-methyl-1,3,4-oxadiazol-2-yl)-3-[(*S*)-methylsulfinyl]-4-(trifluormethyl)benzamid gemäß Anspruch 1 oder eines Mittels gemäß einem der Ansprüche 2 bis 5 auf die unerwünschten Pflanzen und/oder ihren Lebensraum einwirken lässt.

8. Verfahren nach Anspruch 7 zur Bekämpfung von Schadpflanzen in monokotylen Pflanzenkulturen.

9. Verfahren nach Anspruch 7 oder 8, worin die Pflanzenkulturen gentechnisch verändert oder durch Mutationsselektion erhalten sind.
